# EUROPEAN PATENT SPECIFICATION

(11) **EP 4 605 364 B1**
(45) Date of publication and mention of the grant of the patent: **17.06.2026**
(21) Application number: 23789267.4
(22) Date of filing: 09.10.2023
(51) Int. Cl.: C07C 45/00, C07C 45/64, C07F 3/02, C07C 17/263

(54) **PROCESS FOR THE PREPARATION OF 4-CHLORO-2-(TRIFLUOROMETHYL)PHENYL GRIGNARD COMPOUND AND DERIVATIVES THEREOF**
VERFAHREN ZUR HERSTELLUNG VON 4-CHLOR-2-(TRIFLUORMETHYL)PHENYL GRIGNARD-VERBINDUNGEN UND DERIVATEN DAVON
PROCÉDÉ DE PRÉPARATION D'UN COMPOSÉ DE 4-CHLORO-2-(TRIFLUOROMÉTHYL)PHÉNYL- GRIGNARD ET DÉRIVÉS DE CELUI-CI

(30) Priority: 17.10.2022 EP 22201821
(43) Date of publication of application: 27.08.2025
(73) Proprietor: BASF Agro B.V., 6811 AH Arnhem (NL)
(72) Inventor: FRASSETTO, Timo, 67056 Ludwigshafen (DE); ALZNAUER, Christiane, 67056 Ludwigshafen (DE); SAELINGER, Daniel, 67056 Ludwigshafen (DE); VOGT, Florian, 67056 Ludwigshafen (DE)
(74) Representative: BASF IP Association
(86) International application number: PCT/EP2023/077849
(87) International publication number: WO 2024/083545

(56) References cited:
- EP-A1- 0 487 357
- WO-A1-2015/091045
- DATABASE CAPLUS [online] 1 January 2015 (2015-01-01), VOGELBACHER U: "Process for the preparation of substituted phenoxyphenyl ketones - WO2015091045 A1", XP093029374, Database accession no. 2015:1059447
- BOUYSSOU T ET AL: "Discovery of olodaterol, a novel inhaled ^2"2-adrenoceptor agonist with a 24h bronchodilatory efficacy", BIOORGANIC & MEDICINAL CHEMISTRY LETTERS, ELSEVIER, AMSTERDAM NL, vol. 20, no. 4, 15 February 2010 (2010-02-15), pages 1410 - 1414, XP026887080, ISSN: 0960-894X, [retrieved on 20100104], DOI: 10.1016/J.BMCL.2009.12.087
- TISLTAM U: "Activation of Mg Metal for Safe Formation of Grignard Reagents on Plant Scale", ORG. PROC. RES & DEV., 1 January 2002 (2002-01-01), pages 906 - 910, XP093029375, Retrieved from the Internet <URL:https://pubs.acs.org/doi/full/10.1021/op025567%2B> [retrieved on 20230307]
- KOBAYASHI SHOJI ET AL: "Grignard Reactions in Cyclopentyl Methyl Ether", vol. 5, no. 5, 7 March 2016 (2016-03-07), Germany, pages 636 - 645, XP093029376, ISSN: 2193-5807, Retrieved from the Internet <URL:https://api.wiley.com/onlinelibrary/tdm/v1/articles/10.1002%2Fajoc.201600059> DOI: 10.1002/ajoc.201600059

## Description

The present invention relates to a process for providing 4-chloro-2-(trifluoromethyl)phenyl Grignard compound. Further, the present invention relates to the process for preparation of 4-chloro-2-(trifluoromethyl)phenyl compounds from 4-chloro-2-(trifluoromethyl)phenyl Grignard compound, in particular to the process for the preparation of substituted phenoxyphenyl ketones.

4-halo-2-(trifluoromethyl)phenyl Grignard compounds are valuable intermediates for the synthesis of various pharmaceutical and agrochemical compounds as well as polymers. EP 0487357 and Organic Process Research & Development, 2016, vol.20, no. 9, pp.1633-1636 describe the Grignard reactions of halosubstituted trifluoromethylbensenes. Journal of Organic Chemistry, 1976, vol.41, no.23, pp. 3653-3664 describes the directing ability of trifluoromethyl group in metalation reactions.

4-halo-2-(trifluoromethyl)phenyl Grignard compounds are generally prepared starting from 2,5-dihalo trifluoromethylbenzene.

WO2020/157199 describes preparation of a Grignard compound wherein the starting compound has identical halogens in 2- and 5-position. 2,5-dibromo trifluoromethyl benzene undergoes Grignardization in 5-position:

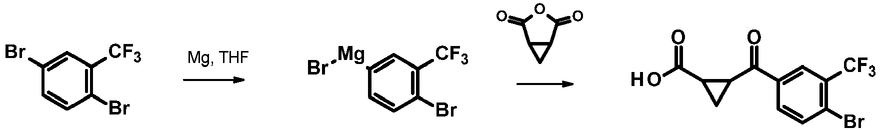

In order to overrule the reported reactivity and selectively allow the Grignard reaction of the halogen in 2-position of a 2,5-dihalo trifluoromethyl benzene, normally a more reactive halogen in the order I > Br > CI > F is introduced in the 2-position of the starting material compared with its 5-position. For example, WO 2015/091045 and Bioorganic and Medicinal Chemistry Letters, 2010, vol. 20, no.4, pp.1410-1414 describe reactions of 2-Br,5-Cl-trifluoromethyl benzene.

The synthesis of a 2,5-dihalo trifluoromethyl benzene with different halogens in 2- and 5-position requires at least two distinct halogenation steps in its production, increasing complexity of the synthesis and typically creating more chemical waste and wastewater. An example for such a synthesis is described in CN104447183. It includes the following steps:

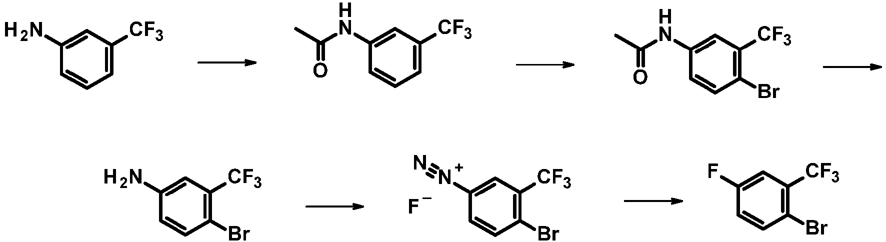

Therefore, there is a continuous need for processes that easily make 4-halo-2-(trifluoromethyl)phenyl Grignard compounds available in good yield employing starting materials that are easily accessible and save resources.

Hence, it was an object of the present invention to develop an efficient and environmentally friendly process for the preparation of 4-halo-2-(trifluoromethyl)phenyl Grignard compounds, leading to the desired product in high yields and being suitable for an upscale to industrially relevant amounts.

It has now surprisingly been found that 2,5-dichloro trifluoromethyl benzene is forming a Grignard compound with solid Mg virtually exclusively in 2-position, which is a complete reversal of the reported regioselectivity of the 2,5-dibromo trifluoromethyl benzene. Furthermore, also the commonly practiced Grignard preparation by trans-grignardisation shows a sluggish conversion with poor selectivity, as demonstrated in comparative Example 12.
this invention

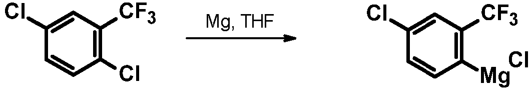

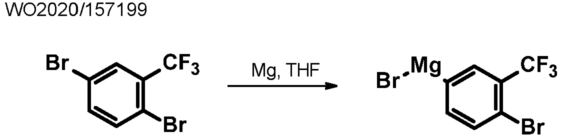

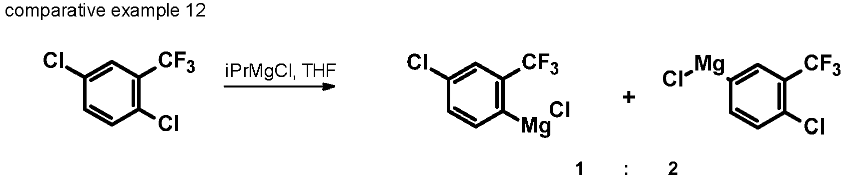

The production process of the starting material 2,5-dichloro trifluoromethyl benzene is short and allows a highly efficient synthesis of 4-chloro-2-(trifluoromethyl) phenyl Grignard compounds. This also enables an efficient synthesis of phenoxyphenyl ketones.

### Grignard compound (I)

In one aspect, the present invention relates thus to a process for the preparation of Grignard compound of formula (I) 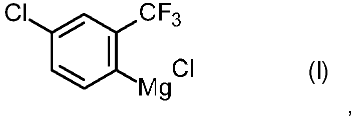 comprising the following steps:
(i) reacting a compound of formula (A) 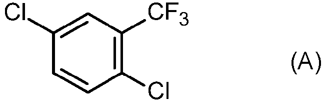 with Mg in the presence of LiCl and a solvent.

In the step (i) the 2,5-dichloro-trifluoromethylbenzene of formula (A) is reacted with magnesium (Mg).

The process according to the present invention entails a series of advantages. It is simple, cheap and leads to the product in high yields. The number of necessary steps towards compound (I) and hence the amounts of wastes are reduced as compared to the processes known from the prior art since the synhesis of the starting 2,5-dichloro trifluoromethylbenzene requires less steps as the synthesis of 2,5-dihalo trifluoromethylbenzene with different halogens. Any of these advantages saves resources and energy and makes the process industrially simple and environmentally friendly.

Further embodiments of the invention are evident from the claims, the description and the examples. It is to be understood that the single features of the subject matter of the invention described herein can be applied not only in the combination given in each particular case but also in other combinations, without leaving the scope of the invention.

Starting compound (A) is commercially available or can be synthesized as known to the skilled person.

Generally, in the step (i) either compound (A) or Mg can be used in excess. Alternatively, the compound (A) and Mg can be used in equimolar amounts.

According to one embodiment, Mg is used in excess. Preferably, 1 to 2 mol, more preferably 1 to 1.5 mol, most preferably 1 to 1.1 mol of Mg in relation to one mol of compound (I) is used.

According to another embodiment, the compound (A) is used in excess. Preferably, 1 to 5 mol, more preferably 1 to 2 mol, most preferably 1 to 1.2 mol of compound (I) in relation to one mol of Mg is used.

According to another embodiment, the compound (A) and Mg are used in equimolar amounts.

The concentration of the Grignard compound in solution after completion of the reaction can vary. Usually, it is up to 3 mol/L, preferably from 0.4 to 3 mol/L, more preferably 0.5 to 2 mol/L, most preferably 0.6 to 1.5 mol/L of compound (I), most preferably 1.0-1.25 mol/L.

According to one embodiment of the inventive process, the Mg surface is activated by using methods or agents known in the art, for example, I₂, alkyl bromides, such as 2-bromopropane, 1,2-dibromoethane; diisobutylaluminum hydride (DIBAH), Red-Al, LiAlH₄, BH₃•SMe₂, NaBH₄. According to a preferred embodiment, 2-bromopropane is used. For further details see, e.g. Asian J. Org. Chem, 2016, 5, 636-45, Org. Proc. Res & Dev. 2002, 906-910.

According to the inventive process, LiCl is added as catalyst to the reaction mixture of step (i). The addition of LiCl leads to higher yield of the Grignard compound (I) and to the less formation of side products. The use of LiCl together with Grignard reagents is generally known in the art, see for example Angew. Chem. Int. Ed. 2004, 43, 3333 and Angew. Chem. Int. Ed. 2006, 45, 159. Preferably, 0.01 to 0.5 mol, more preferably 0.01 to 0.3 mol, most preferably 0.01 to 0.1 mol of LiCl in relation to one mol of compound (I) is used.

Suitable solvents for the step (i) are, for example, aliphatic, alicyclic or aromatic hydrocarbons such as hexane, heptane, cyclohexane, toluene, o-, m- and p-xylenes, mesitylene, ethers such as ethyl propyl ether, tert-amyl methyl ether, methyl tert-butyl ether, n-butyl methyl ether, anisole, phenetole, cyclohexyl methyl ether, cyclopentyl methyl ether, dimethyl ether, diethyl ether, dimethyl glycol diphenyl ether, dipropyl ether, diisopropyl ether, di-n-butyl ether, diisobutyl ether, diisoamyl ether, ethylene glycol dimethyl ether, bis(2-methoxyethyl) ether, isopropyl ethyl ether, tetrahydrofuran, methyltetrahydrofuran, dioxane and polyethers of ethylene oxide and/or propylene oxide. In one embodiment, preferable solvent can be selected from tetrahydrofuran (THF), 2-methyl-tetrahydrofuran, 1,4-dioxane, diethyl ether, methyl-tert-butylether (MTBE), toluene, m-xylene, o-xylene, p-xylene, or any mixture thereof. In another embodiment, the preference is given to using tetrahydrofuran, methyltetrahydrofuran, methyl tert-butyl ether ether and mixtures of tetrahydrofuran, methyltetrahydrofuran, methyl tert-butyl ether with toluene and/or xylenes. Specifically, THF is preferred.

The step (i) is generally carried out under atmospheric pressure. However, it is also possible as an alternative to work under reduced pressure or under superatmospheric pressure. According to one embodiment, the reaction can be carried out under inert gas, such as nitrogen or argon, atmosphere. The reaction time is not critical and can be appropriately selected depending on the batch size and on the temperature.

During reaction step (i), the temperature is preferably held at a maximum of 120°C, more preferably at a maximum of 80°C, most preferably at a maximum of 45°C, particularly preferable at maximum of 30°C. Generally, it is preferred to have a reaction temperature of -80°C to 120°C, in particular -40°C to 80°C, especially -5°C to 45 °C. In a further embodiment, the temperature is 10°C to 30°C.

As generally known to the skilled person, the structure of a Grignard reagent can be described by the so-called Schlenck equilibrium. A Grignard reagent undergoes a solvent-dependent equilibrium between different magnesium compounds. The Schlenck equilibrium for the Grignard reagent used according to the present invention can be schematically illustrated as follows: 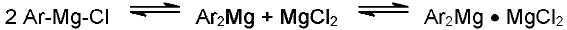 wherein Ar stangs for

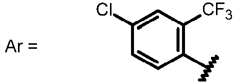

Furthermore, it is known, that solvent molecules, in particular ethers such as diethylether or THF, which are commonly used for reactions with Grignard reagents, can add to the magnesium of the Grignard reagent thereby forming etherates. It is apparent fo the skilled person that also other solvent molecules may be present, depending on the solvent used in the reaction. For general information regarding structures of Grignard reagents, see also Milton Orchin, Journal of Chemical Education, Volume 66, Number 7, 1999, pp 586 to 588.

Examples of the species which can be found in equilibrium including etherates in the case of THF are depicted below:

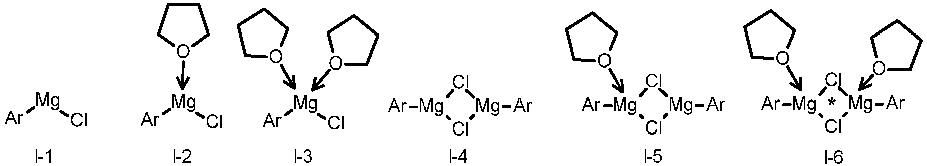

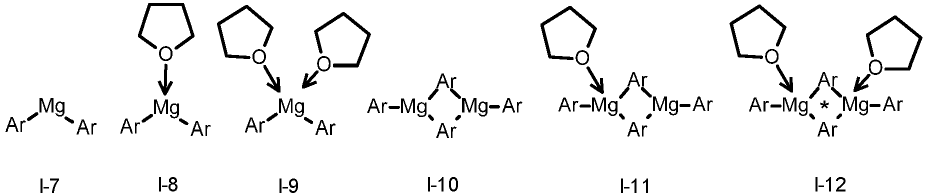

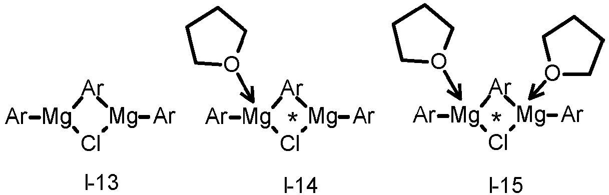

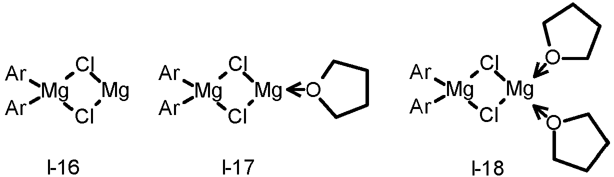

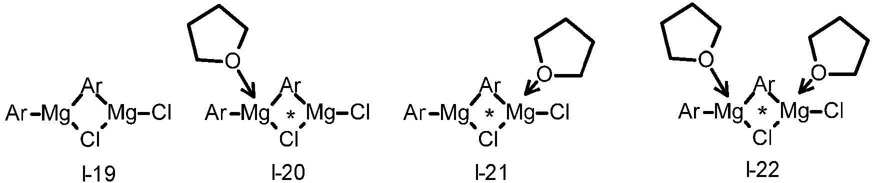

Note 1: Ar or Cl, having two bonds stands for a three-center-two-electron-bond.

Note 2: If Mg carries four substituents, it is coordinated tetrahedrally. Thereby, depending on the specific structure, stereoisomers (diastereomers and/or enantiomers) may occur (marked with *). This is demonstrated on a specific example as follows:

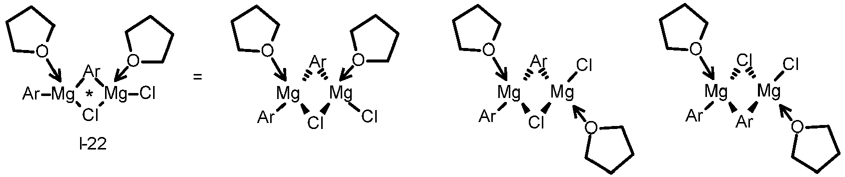

The different magnesium compounds occurring in the inventive process, in particular of the kind as shown above, and possible adducts with solvent molecules are also an aspect of the present invention.

### 4-chloro-2-(trifluoromethyl)phenyl compounds

In another embodiment of the present invention, the Grignard compound of formula (I) is used for the synthesis of 4-chloro-2-(trifluoromethyl)phenyl compounds.

Thus, in another aspect, the present invention relates to a process for the preparation of compounds of formula (II) 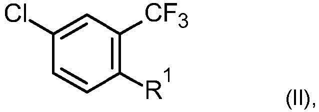 wherein:
R¹ is selected from -CR^{a}R^{b}OH, -C(O)R^{c}, -B(OH)₂, B(OC₁-C₄-alkyl)₂;
R^{a} and R^{b} are independently selected from H or C₁-C₄-alkyl or phenyl, wherein phenyl can be unsubstituted or bear 1, 2 or 3 substituents selected from halogen or C₁-C₆-alkyl; or
R^{a} and R^{b} together with the C-atom to which they are attached form a C₃-C₆-cycloalkyl ring;
R^{c} is selected from H, OH or C₁-C₄-alkyl or phenyl, wherein phenyl can be unsubstituted or bear 1, 2 or 3 substituents selected from halogen or C₁-C₆-alkyl;
comprising the following steps:
   (i) preparing the compound of formula (I) 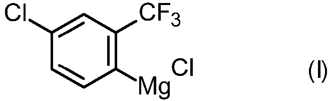
      by reacting a compound of formula (A) 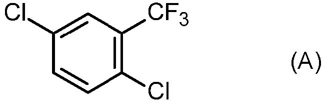
      with Mg in the presence of LiCl and a solvent;
   (ii) reacting the compound of formula (I) with an electrophile (E).

As electrophile any suitable electrophile can be used. Examples of suitable electrophiles are

(E1) R^{a}C(O)R^{b},

(E2) R^{c}C(O)X,

(E3) B(OC₁-C₄-Alkyl)₃,

(E4) CO₂,

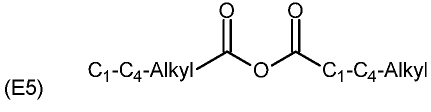

or

(E6) R^{c}CN

wherein
R^{a} and R^{b} are independently selected from H or C₁-C₄-alkyl, or phenyl, wherein phenyl can be unsubstituted or bear 1, 2 or 3 substituents selected from halogen or C₁-C₆-alkyl; or
R^{a} and R^{b} together with the C-atom to which they are attached form a C₃-C₆-cycloalkyl ring;
R^{c} is selected from H, C₁-C₄-alkyl or phenyl, wherein phenyl can be unsubstituted or bear 1, 2 or 3 substituents selected from halogen or C₁-C₆-alkyl;
X is selected from halogen or -NR^{a}R^{b}, -N(C₁-C₄-alkyl)-O-(C₁-C₄-alkyl), N-morpholino.

The term "halogen" refers to fluorine, chlorine, bromine and iodine.

The term "C₁-C₆-alkyl" refers to a straight-chained or branched saturated hydrocarbon group having 1 to 6 carbon atoms, e.g. CH₃, C₂H₅, n-C₃H₇, CH(CH₃)₂, n-butyl, iso-butyl and tert-butyl, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. A preferred embodiment of a C₁-C₆-alkyl is a C₂-C₄-alkyl. Likewise, the term "C₁-C₄-alkyl" refers to a straight-chained or branched alkyl group having 1 to 4 carbon atoms, e.g. CH₃, C₂H₅, n-C₃H₇, CH(CH₃)₂, n-butyl, iso-butyl and tert-butyl.

The term "C₃-C₆-cycloalkyl" refers to monocyclic saturated hydrocarbon radicals having 3 to 6 carbon ring members, such as cyclopropyl, cyclobutyl, cyclopentyl, or cyclohexyl.

In one embodiment of the present invention the electrophile is (E1) R^{a}C(O)R^{b}, for example an aldehyde, such es formaldehyde or acetic acid aldehyde, or a ketone, for example acetone, methylethyl ketone.

In another embodiment of the present invention the electrophile is (E2) R^{c}C(O)X, for example acid halides, such as acetyl chloride (AcCl).

In another embodiment of the present invention the electrophile is (E3) B(OC₁-C₄-alkyl)₃.

In another embodiment of the present invention the electrophile is (E4) CO₂.

In another embodiment of the present invention the electrophile is (E5) 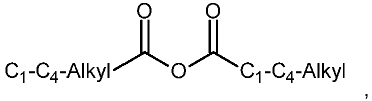 for example acetic acid anhydride (Ac₂O).

In another embodiment of the present invention the electrophile is (E6) R^{c}CN, for example alkyl cyanide such as (C₁-C₄-alkyl)CN.

The electrophile (E) is preferably used in an equimolar amount or in excess as compared to the Grignard compound (I). The excess depends on the electrophile used. For examples, the electrophil selected from (E1), (E2), (E3), (E5) or (E6), is used in an amount of 1 to 3 mol, preferably 1 to 2.5 mole, more preferably 1 to 2 mol, in relation to one mole of of compound (I). In particular the amounts of 1 to 1.5 mole, more specifically 1.05 to 1.1 mole per mole of compound (I) may be favorable according to the present invention. The electrophil (E4) is usually passed through the reaction mixture until all Grignard compound (I) has been converted.

Suitable solvents for the step (ii) are, for example, aliphatic, alicyclic or aromatic hydrocarbons such as toluene, o-, m- and p-xylenes, mesitylene, ethers such as ethyl propyl ether, tert-amyl methyl ether, methyl tert-butyl ether, n-butyl methyl ether, anisole, phenetole, cyclohexyl methyl ether, cyclopentyl methyl ether, dimethyl ether, diethyl ether, dimethyl glycol diphenyl ether, dipropyl ether, diisopropyl ether, di-n-butyl ether, diisobutyl ether, diisoamyl ether, ethylene glycol dimethyl ether, isopropyl ethyl ether, tetrahydrofuran, methyltetrahydrofuran, dioxane, and polyethers of ethylene oxide and/or propylene oxide. In one embodiment, preferable solvent can be selected from tetrahydrofuran (THF), 2-methyl-tetrahydrofuran, 1,4-dioxane, diethyl ether, methyl-tert-butylether (MTBE), toluene, m-xylene, o-xylene, p-xylene, or any mixture thereof. In another embodiment, the preference is given to using tetrahydrofuran, methyltetrahydrofuran, methyl tert-butyl ether ether and mixtures of tetrahydrofuran, methyltetrahydrofuran, methyl tert-butyl ether with toluene and/or xylenes. Specifically, THF is preferred.

The step (ii) is generally carried out under atmospheric pressure. However, it is also possible as an alternative to work under reduced pressure or under superatmospheric pressure.

The reaction time is not critical and can be appropriately selected depending on the batch size and on the temperature.

The order of adding reagents to the reaction mixture is variable. Compound (I) and electrophile (E) can be added simultaneously (in parallel) or in succession. According to one embodiment they are added in parallel. According to another embodiment, they are added in succession. In this case, the order of adding is not critical. Either compound (I) or electrophile (E) can be added at first. Alternatively, compound (I) and a part of electrophile (E) are added in parallel to the remaining part of electrophile (E).

The reaction temperature in step (ii) is preferably held at a maximum of 70°C, in particular at a maximum of 50 °C, more preferably at a maximum of 35°C. Generally, it is preferred to have a reaction temperature of -78°C to 70 °C, in particular -78°C to 50°C, in particular -50°C to 25°C. In a further embodiment, the temperature is 0°C to 15°C, specifically 0°C to 5°C.

In the further course of reaction step (ii), the temperature is preferably held at a maximum of 70°C, in particular at a maximum of 50°C, more preferably at a maximum of 45°C. Generally, it is preferred to have a reaction temperature of 30°C to 50°C, in particular 35°C to 45°C. In a further embodiment, the temperature is 20°C to 35°C, specifically 25°C to 30°C.

According to one embodiment, the step (ii) can be carried out in the presence of a Cu(I)-catalyst, for example a Cu(I) salt or Cu(I) oxide, in particular a Cu(I) salt such as Cu(I)Cl or Cu(I)Br or any mixture thereof. According to one specific embodiment, Cu(I)Cl is used. Preferably, 0.01 to 0.5 mol, more preferably 0.01 to 0.2 mol, most preferably 0.01 to 0.05 mol, especially preferably 0.1 to 0.02 mol of a Cu(I)-catalyst in relation to one mol of compound (I) is used.

The Grignard compound (I) is added in the manner as is common to the skilled person. In particular, the solution obtained in step (i) can be directly used in step (ii). If Mg is used in excess, undissolved Mg-particles can be removed prior to use. These removed Mg particles can be used in the next batch.

### 4-chloro-1-[4-chloro-2-(trifluoromethyl)phenyl]-2-(trifluoromethyl)benzene

In another aspect, the present invention relates to a process for the preparation of 4-chloro-1-[4-chloro-2-(trifluoromethyl)phenyl]-2-(trifluoromethyl)benzene (III) 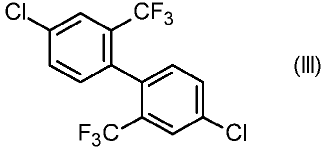 comprising the following steps
(i) preparing the compound of formula (I) 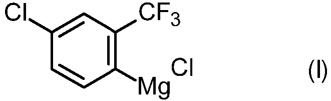
   by reacting a compound of formula (A) 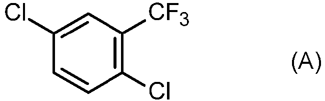
   with Mg in the presence of LiCl and a solvent;
(iii) dimerizing the compound of formula (I), optionally in the presence of a catalyst.

In the step (iii) the catalyst can be used. It is preferably selected from iron, copper, cobalt, manganese, nickel, zinc, titanium, vanadium and ruthenium catalysts.

According to one ambodiment, step (iii) is carried out in the presence of a terminal oxidizing agent preferably selected from dioxygen, a diaziridinone, or 1,2-dihaloethane.

Suitable solvents for the step (iii) are those suitable for step (i).

The step (iii) is generally carried out under atmospheric pressure. However, it is also possible as an alternative to work under reduced pressure or under superatmospheric pressure.

The reaction time is not critical and can be appropriately selected depending on the batch size and on the temperature.

### Phenoxyphenyl ketones

In another aspect, the present invention relates to a process for the preparation of phenoxyphenyl ketones of formula (IV) 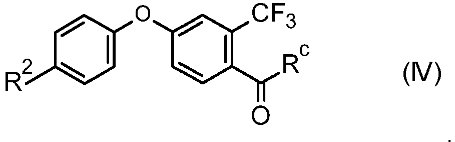 Wherein
- R^{c}: is C₁-C₄-alkyl;
- R²: is F or Cl;
comprising the following steps:
(i) preparing the compound of formula (I) 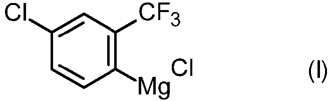
   by reacting a compound of formula (A) 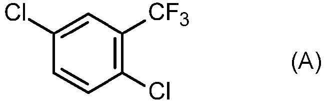
   with Mg in the presence of LiCl and a solvent;
(ii) preparing the compound of formula (II-A), 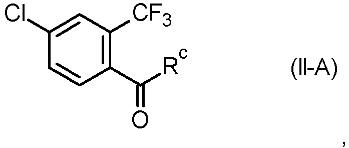 by reacting compound of formula (I) with an electrophile selected from

   (E2-1) R^{c}C(O)Hal, (E2-2) R^{c}C(O)N(C₁-C₄-alkyl)-O-(C₁-C₄-alkyl) or

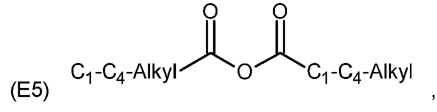

   or

   (E6) R^{c}CN

   wherein
   R^{c} is C₁-C₄-alkyl, and
   Hal is halogen
(iv) reacting compound (II-A) with a phenol derivative of formula (V) 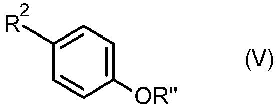
   in the presence of a base if R" is hydrogen;
   wherein the variables are defined as follows:
      - R²: is F or Cl;
      - R": is hydrogen, N(C₁-C₈-alkyl)₄ cation or an alkali metal cation.

The compound (II-A) can be used directly from step (ii) without further purification or can be used in purified form.

Examples for appropriate solvents for step (iv) are aprotic organic solvents such as for example dimethyl formamide (DMF), dimethyl acetamide (DMAC), N-methyl pyrrolidone (NMP), dimethyl imidazolidinone (DMI), tetramethylurea (TMU), N,N'-dimethylpropyleneurea (DMPU), dimethylsulfoxide (DMSO), toluene, o-xyleneand any mixtures thereof. In particular DMF, DMAC, NMP and toluene or any mixtures, more specifically DMF, DMAC and NMP are particularly suitable.

The base used in step (iv) is preferably an inorganic base, according to one embodiment selected from NaOH, KOH, Na₂CO₃, K₂CO₃, Na₃PO₄ and K₃PO₄. According to one embodiment NaOH is used. Acording to another embodiment KOH is used. Acording to still another embodiment Na₂CO₃ is used. Acording to still another embodiment K₂CO₃ is used. Acording to still another embodiment Na₃PO₄ is used. According to still another embodiment K₃PO₄ is used.

The base can be used in solid form or as a solution, e.g. as aqueous solution.

The reagents for step (iv) are preferably added at ambient temperature and the reaction temperature is then elevated, wherein the reaction temperature after the reagents have been added is preferably held at a maximum of 160°C, in particular at a maximum of 145°C, more preferably at a maximum of 140°C. Generally, it is preferred to have a reaction temperature of 20°C to 160°C, in particular 50°C to 150°C, more particularly 100°C to 140°C.

After step (iv), a work-up of the reaction mixture can be carried out by procedures known in a general manner to the person skilled in the art. Generally, water is added and the aqueous phase is extracted with a suitable solvent, e.g. toluene or o-xylene. The raw product obtained after evaporation of the solvent(s) can directly be used in a further step, if desired. However, the raw product can also be further worked up and/or purified as generally known to the skilled person.

Each step (i), (ii), (iii) or (iv)can be carried out batchwise or continuously.

### Examples

The following examples further illustrate the present invention and do not restrict the invention in any manner.

### Example 1: Synthesis of the Grignard compound (I)

0.240 g (10.0 mmol) magnesium turnings were placed in a flask and 2 g THF were added followed by 120 mg (2.8 mmol) LiCl. 60 mg (0.5 mmol) 2-bromopropane were added to activate the magnesium. After 5 min, 2.0 g (9.3 mmol) 2,5-dichloro-trifluoromethylbenzene in 4 ml THF were added and the temperature was allowed to reach 50 °C. The mixture was post-stirred for 1 h at 40 °C. The mixture was cooled to 5 °C and 0.4 g methanol was added. Water and tert-butyl methyl ether (MTBE) was added followed by 32% HCl. The organic phase was concentrated and analyzed by GC to show a ratio of 3-chloro-trifluoromethylbenzene / 2-chloro-trifluoromethylbenzene of 30:1.

GC-method: Agilent Technologies 7890A, column DB-XLB 30x250 µm x 1µm, inlet 280 °C, detector 320 °C, constant flow 1.145 mL/min He, split ratio 50, 0 min: 55 °C, 2 min: 55 °C, 27.5 min: 310 °C, 40 min: 310 °C.

Retention times: 3-chloro-trifluoromethylbenzene 9.0 min and 2-chloro-trifluoromethylbenzene 10.3 min.

### Example 2: Synthesis of the Grignard compound (I)

13.5 g (0.56 mol, 1.1 eq) Mg turnings were placed in a reaction vessel followed by 6.3 g LiCl (0.15 mol, 30 mol%). 80 g THF was added. 3.1 g (25 mmol, 5 mol%) 2-bromopropane was added to initiate the Grignard formation. 10 min after start of the exothermic reaction, a solution of 109 g (99%, 0.50 mol, 1.0 eq) 2,5-dichloro-trifluoromethylbenzene in 600 g THF was being added within 3 h at a constant reaction temperature of 25 °C. The mixture was post-stirred for 1 h at 25 °C to complete the Grignard formation. The most part of the formed Grignard solution (765 g) was transferred via a canula into a storage bottle. Excess Mg remained in the reaction vessel together with approximately 50 g of the Grignard-solution. 50 g of fresh THF was added to dilute the hold-up in the reaction vessel.

### Example 3: Synthesis of the Grignard compound (I)

12.2 g Mg-turnings (0.50 mol, 1.0 eq) and 2.2 g LiCl (52 mmol, 0.1 eq) were added to the Mg hold-up of example 2. A solution of 109 g (99%, 0.50 mol, 1.0 eq) 2,5-dichloro trifluoromethylbenzene in 600 g THF was added within 3 h at a constant reaction temperature of 25 °C. The mixture was post-stirred for 1 h at 25 °C to complete the Grignard formation. The most part of the formed Grignard solution was transferred via a canula into a storage bottle. Excess Mg remained in the reaction vessel together with approximately 50 g of the Grignard-solution. 50 g of fresh THF is added to dilute the hold-up in the reaction vessel.

### Example 4: Reaction of the Grignard compound (I) with acetyl chloride

4.0 g (0.05 mol) acetyl chloride was diluted with 120 g toluene. 2.5 g (25 mmol) copper(I)chloride was added, and the temperature was adjusted to 0 °C. In parallel, a solution of 41.7 g (0.53 mol) acetyl chloride in 120 g toluene and 765 g Grignard solution (from example 2) was added. The reaction temperature was kept at 0-2 °C during the addition. The mixture is post-stirred for 60 min at 0 °C to complete the acylation. 245 g water were added within 15 min and the phases were separated. The organic phase was washed with 150 g 2.5% HCl followed by 115 g 3% NH3-solution. The organic phase was concentrated to 102 g oily residue containing 83.3% 1-[4-chloro-2-(trifluoromethyl)phenyl]ethanone by quantitative GC. The yield was 0.38 mol.

¹H-NMR (400 MHz, CDCl₃): 2.56 (s, 3H), 7.43 (d, *J* = 8.3 Hz, 1H), 7.58 (dd, *J* = 8.3, 1.9 Hz, 1 H), 7.70 (d, *J* = 2.0 Hz, 1H).

### Example 5: Reaction of the Grignard compound (I) with cyclohexanone

2.8 g (28 mmol) cyclohexanone in 8 ml THF were added to 50 g of the prepared Grignard solution (from example 2) at 0 °C. The mixture was stirred at 0 °C for 1 h followed by 2 h at 23 °C. Saturated NH₄Cl and water (to dissolve precipitated salts) were added and the product was extracted with MTBE. The solvent was removed under reduced pressure delivering 4.0 g oily residue. The product 1-[4-chloro-2-(trifluoromethyl)phenyl]cyclohexanol was identified by NMR.

¹H-NMR (400 MHz, DMSO-d₆): 1.16-1.29 (m, 1H), 1.42-1.67 (m, 4 H), 1.70-1.82 (m, 5H), 4.81 (s, 1H), 7.64 (dd, *J* = 8.8,2.2 Hz, 1H), 7.71 (d, *J* = 2.3 Hz, 1 H), 7.73 (d, *J* = 8.8 Hz, 1H).

### Example 6: Reaction of the Grignard compound (I) with trimethyl borate

3.0 g trimethyl borate (29 mmol) was dissolved in 8 g THF. 50 g of the Grignard solution (from example 2) were added at 0 °C. The mixture was warmed to 25 °C and stirred for 2 h. 14 g HCl 10% was added and the mixture was stirred at 50 °C for 2 h. The aqueous phase was removed. The organic phase was washed with water and the combined aqueous phases were extracted with MTBE. The combined organic phases were concentrated to 5.0 g organic residue. The product [4-chloro-2-(trifluoromethyl)phenyl]boronic acid was identified by NMR.

¹H-NMR (400 MHz, DMSO-d₆): 4.25 (br, 1H), 7.58 (d, *J* = 6.4 Hz, 1H), 7.68 (d, *J* = 6.4 Hz, 1 H), 7.73 (s, 1H), 8.34 (br, 1H).

### Example 7: Reaction of the Grignard compound (I) with carbon dioxide

50 g of the Grignard solution (from example 2) were cooled to 0 °C and CO₂ was bubbled through the solution until the exotherm reaction had subsided. Additional CO₂ was passed through the solution for 1h, 5 g water was added followed by 20 g HCl 5%. The mixture was warmed to 70 °C, the aqueous phase was removed and the organic phase washed with water. The combined aqueous phases were extracted with MTBE and the combined organic phases concentrated under reduced pressure to give 7.0 g residue. The product 4-chloro-2-(trifluoromethyl)benzoic acid was identified by NMR.

¹H-NMR (400 MHz, DMSO-d₆): 7.85 (d, *J =* 8.8 Hz, 1H), 7.89 (s, 1 H), 7.73 (s, 1H), 7.92 (d, *J* = 8.3 Hz, 1H), 13.2 (br,1H).

### Example 8: Reaction of the Grignard compound (I) with DMF

50 g of the Grignard solution (from example 2) was cooled to -72 °C and 4.0 g DMF (0.54 mol) was added at -72 °C. The solution was slowly warmed to 0 °C and post-stirred for 1 h at 0 °C. Saturated KH₂PO₄-solution was added followed by 5 %HCl to dissolve the precipitated salts. The mixture was extracted with MTBE and the organic phase was concentrated under reduced pressure to yield 3.0 g of a brown oil. The product 4-chloro-2-(trifluoromethyl)benzaldehyde was identified by NMR.

¹H-NMR (400 MHz, DMSO-d₆): 7.94-8.02 (m, 2H), 8.13 (d, *J =* 8.3 Hz, 1H), 10.26 (s, 1H).

### Example 9: Synthesis of the Grignard compound (I) and reaction with acetic anhydride

14.5 g (0.60 mol, 1.2 eq) Mg turnings were placed in a reaction vessel followed by 6.2 g LiCl (0.15 mol, 30 mol%). 80 g THF was added. 3.1 g (25 mmol, 5 mol%) 2-bromopropane was added to initiate the Grignard formation. The start of the reaction was accompanied by an increase of the temperature. 10 min after the start of the exothermic reaction, a solution of 109 g (99%, 0.50 mol, 1.0 eq) 2,5-dichloro-trifluoromethylbenzene in 600 g THF was added within 3 h at a constant reaction temperature of 25 °C. The mixture was post-stirred for 1 h at 25 °C to complete the Grignard formation. The most part of the formed Grignard solution (785 g) was transferred via a canula and discarded. Excess Mg remained in the reaction vessel together with approximately 50 g of the Grignard-solution. 50 g of fresh THF was added to dilute the hold-up in the reaction vessel.

12.2 g Mg-turnings (0.50 mol, 1.0 eq) and 2.2 g LiCl (52 mmol, 0.1 eq) were added to the hold-up of the first batch. A solution of 109 g (99%, 0.50 mol, 1.0 eq) 2,5-dichloro-trifluoromethylbenzene in 600 g THF was being added within 3 h at a constant reaction temperature of 25 °C. The mixture was post-stirred for 1 h at 25 °C to complete the Grignard formation. The formed Grignard solution was transferred via a canula into a storage bottle (755 g). Excess Mg remained in the reaction vessel together with approximately 50 g of the Grignard-solution. 50 g of fresh THF was added to dilute the hold-up in the reaction vessel.

2.5 g acetic anhydride (25 mmol, 0.05 eq) was mixed with 120 g THF and cooled to -5°C. Within 3 h, the Grignard solution above and in parallel 51.5 g acetic anhydride (0.50 mol, 1.0 eq) were added via syringe pumps keeping the temperature at maximum 2 °C. The reaction mixture was post-stirred for 1 h at 0 °C after the additions were complete. 260 g water were added followed by 3.8 g concentrated HCl to adjust the pH to 7. The biphasic mixture was stirred for 30 min and the aqueous phase was removed. The organic phase was concentrated to 107 g brown oil with a purity of 89% by GC of the desired product 1-[4-chloro-2-(trifluoromethyl)phenyl]ethanone. The chemical yield was 85%.

The impurity1-[4-chloro-3-(trifluoromethyl)phenyl]ethanone, resulting from a displacement of the undesired chloro substituent in step (i) and reaction with acetic anhydride, was detected with <1% proving the extraordinary selectivity in step (i).

### Example 10: Dimerization

50 g of the Grignard solution (from example 2) were mixed with 20 g toluene and 0.24 g CuCl (2.4 mmol). This mixture was stirred for 4 h in a flask open to the atmosphere. The internal temperature peaked at 35 °C after 30 min before dropping down to 23 °C again. 5% aqueous HCl was added until all solids had dissolved. The phases were separated, and the organic phase was concentrated under reduced pressure to obtain 5.0 g of a brown oil. The product 4-chloro-1-[4-chloro-2-(trifluoromethyl) phenyl]-2-(trifluoromethyl)benzene was identified by NMR in agreement with the literature values (Liebigs Ann. 1995, 5, 781-6).

¹H-NMR (400 MHz, MeOD): 7.34 (d, *J* = 8.6 Hz, 2H), 7.68 (dd, *J* = 8.6, 2.2 Hz, 2H), 7.82 (d, *J* = 2.2 Hz, 2H).

### Example 11: Synthesis of 1-[4-(4-chlorophenoxy)-2-(trifluoromethyl)phenyl]ethanone

117 g p-chlorophenol (0.91 mol, 2.0 eq) were dissolved in 668 g NMP. 36.4 g solid KOH (90%, 0.58 mol, 1.3 eq) was added and the temperature raised to 100 °C until all KOH was dissolved. 104.2 g (96%, 0.45 mol, 1.0 eq) molten 1-[4-chloro-2-(trifluoromethyl)phenyl]ethenone was added via a dropping funnel. 223 g NMP was used to wash all acetophenone into the reaction flask. The temperature was increased gradually to 145 °C and the mixture stirred for 14 h to complete the reaction. NMP was removed by distillation. 500 g toluene and 1400 g water were added. 207 g 10% aqueous NaOH (0.52 mol, 1.15 eq) was added and the phases separated. The aqueous phase was extracted twice with 500 g toluene. The combined organic phases were washed with 450 g water and 45 g 10% NaOH (0.11 mol, 0.25 eq). The phases were separated. The organic phase was concentrated under reduced pressure to yield 141 g oily product (88% by HPLC, 0.39 mol, 88% chemical yield).

¹H-NMR (400 MHz, CDCl₃): 2.57 (s, 3H), 6.98-7.03 (m, 2H), 7.12 (dd, *J* = 8.8, 2.5 Hz, 1 H), 7.31 (d, *J* = 2.2 Hz, 1H), 7.35-7.40 (m, 2H), 7.50 (d, *J* = 8.4 Hz, 1 H).

### Example 12: Comparative example trans-Grignard:

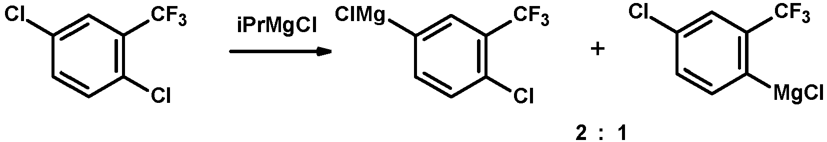

2.1 g (10 mmol) 2,5-dichloro-trifluoromethylbenzene were dissolved in 15 g THF. 0.2 g (5 mmol) LiCl was added, and the mixture was warmed to 30 °C. 5.6 mL isopropylmagnesium chloride solution (2 M in THF, 11 mol) was added within 30 min. The mixture was stirred for 3 h at 30 °C and left standing over night. It was reheated to 50 °C for 2 h to improve conversion. Toluene and 10% aqueous HCl was added, the phases separated, and the organic phase analyzed by GC. The GC showed 77% starting material, 3% 3-chloro-trifluoromethylbenzene and 6% 2-chloro-trifluoromethylbenzene (by area).

### Example 13: Synthesis of the Grignard compound (I) in absence of LiCl, reaction with acetic anhydride for analytical purposes:

14.6 g Mg-turnings (0.60 mol, 1.2 eq) were suspended in 135 mL THF. 10 g Isopropylmagnesium chloride (2.0 M in THF, 0.975 g/ml) were added to consume all residual water. 2.2 g (18 mmol) 2-Brompropane was added to activate the magnesium, showing an exotherm to 31 °C. When a small amount of 2,5-dichloro trifluoromethylbenzene was added, no exotherm could be observed. Another 1.5 g (12 mmol) 2-bromopropane was added to ensure a smooth Grignard formation. A solution of 109 g (99%, 0.50 mol, 1.0 eq) 2,5-dichloro trifluoromethylbenzene in 203 g THF was added within 4 h at a constant reaction temperature of 25-27 °C. The mixture was post-stirred for 1 h at 25 °C. The most part of the formed Grignard solution was transferred via a canula into a storage bottle. Excess Mg remained in the reaction vessel together with approximately 50 g of the Grignard-solution.

A small sample was brought to reaction with excess acetic anhydride to control the quality of the Grignard solution by GC. The GC revealed the presence of several impurities in unusually high amounts that are formed by the reaction of (I) with starting material (A) when there is accumulation of (A) during Grignard formation:
3-chlorobenzotrifluoride: 17.5 area% (typical value <10% if 10 mol% LiCl is present during Grignard formation)
1-[4-chloro-2-(trifluoromethyl)phenyl]ethanone: 62.1 area% (15.6 min, typical value >75%)
1-[2,5-dichloro-3-(trifluoromethyl)phenyl]ethanone: 5.7 area% (17.5 min, typical value <2%)
1-[2,5-dichloro-4-(trifluoromethyl)phenyl]ethanone: 6.5 area% (17.7 min, typical value <2%) (THF is neglected in area% calculation)

## Claims

1. A process for preparation of the Grignard compound of formula (I) 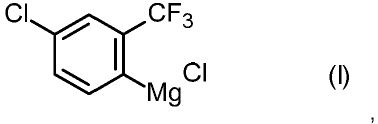 comprising the following steps:
(i) reacting a compound of formula (A) 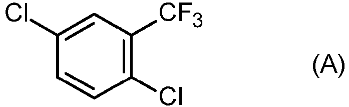 with Mg in the presence of LiCl and a solvent.

2. The process of claim 1, wherein the Mg is activated.

3. The process of claim 2, wherein the magnesium activator is 2-bromopropane.

4. The process of any one of claims 1 to 3, wherein the solvent is selected from aliphatic, alicyclic or aromatic hydrocarbons, ethers and polyethers of ethylene oxide and/or propylene oxide.

5. The process of any one of claims 1 to 4, wherein the solvent is selected from hexane, heptane, cyclohexane, tetrahydrofuran (THF), 2-methyl-tetrahydrofuran, 1,4-dioxane, diethyl ether, methyl-tert-butylether (MTBE), toluene, m-xylene, o-xylene, p-xylene, or any mixture thereof.

6. The process of any one of claims 1 to 5, wherein the solvent is THF.

7. A process for preparation of compounds of formula (II) 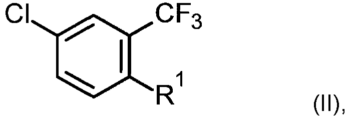 wherein:
R¹ is selected from -CR^{a}R^{b}OH, -C(O)R^{c}, -B(OH)₂, B(OC₁-C₄-Alkyl)₂
R^{a} and R^{b} are independently selected from H or C₁-C₄-alkyl or phenyl, wherein phenyl can be unsubstituted or bear 1, 2 or 3 substituents selected from halogen or C₁-C₆-alkyl; or
R^{a} and R^{b} together with the C-atom to which they are attached form a C₃-C₆-Cycloalkyl ring;
R^{c} is selected from H, OH or C₁-C₄-alkyl or phenyl, wherein phenyl can be unsubstituted or bear 1, 2 or 3 substituents selected from halogen or C₁-C₆-alkyl;
comprising the following steps:
(i) preparing the compound of formula (I) according to the process as described in claim 1, 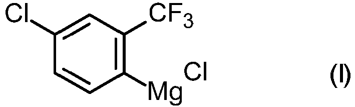
(ii) reacting the compound of formula (I) with an electrophile, wherein the electrophile is selected from
(E1) R^{a}C(O)R^{b},
(E2) R^{c}C(O)X,
(E3) B(OC₁-C₄-Alkyl)₃,
(E4) CO₂, 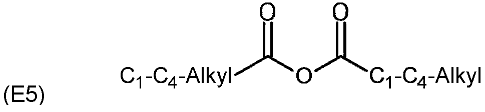
or
(E6) R^{c}CN
wherein
R^{a} and R^{b} are independently selected from H or C₁-C₄-alkyl, or phenyl, wherein phenyl can be unsubstituted or bear 1, 2 or 3 substituents selected from halogen or C₁-C₆-alkyl; or
R^{a} and R^{b} together with the C-atom to which they are attached form a C₃-C₆-Cycloalkyl ring;
R^{c} is selected from H, C₁-C₄-alkyl or phenyl, wherein phenyl can be unsubstituted or bear 1, 2 or 3 substituents selected from halogen or C₁-C₆-alkyl;
X is selected from halogen or -N(C₁-C₄-alkyl)₂, -N(C₁-C₄-alkyl)-O-(C₁-C₄-alkyl), N-morpholine.

8. The process of claim 7, wherein the electrophile is selected from
(E2-1) R^{c}C(O)X,
wherein X represents a halogen,
(E2-2) R^{c}C(O)N(C₁-C₄-alkyl)-O-(C₁-C₄-alkyl), 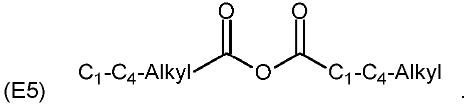
or
(E6) R^{c}CN.

9. The process of claim 8, wherein the electrophile is selected from acetyl chloride (AcCl) or acetic acid anhydride (Ac₂O).

10. The process of any one of the claims 7 to 9, wherein the reaction is carried out in the presence of a Cu-catalyst.

11. A process for preparation of a compound of formula (III) 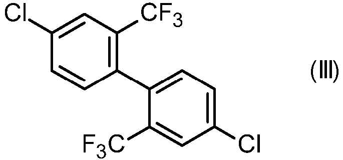 comprising the following steps:
(i) preparing the compound of formula (I) according to the process as described in claim 1, 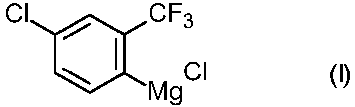
(iii) dimerizing the compound of formula (I).

12. The process of claim 11, wherein the dimerization is carried out in the presence of a catalyst.

13. The process of claim 11 or 12, wherein the reaction is carried out in the presence of a terminal oxidizing agent selected from dioxygen, a diaziridinone, or 1,2-dihaloethane.

14. A process for preparation of compound of formula (IV) 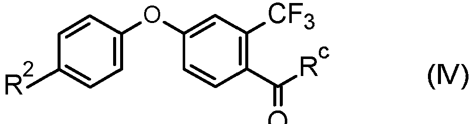 wherein
R^{c} is C₁-C₄-alkyl;
R² is F or Cl;
comprising the following steps:
(i) preparing the compound of formula (I) according to the process as described in claim 1, 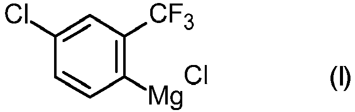
(ii) preparing the compound of formula (II-A) according to the process as described in claim 8, 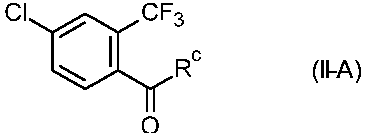 wherein R^{c} is C₁-C₄-alkyl,
(iv) reacting compound (II-A) with a phenol derivative of formula (V) 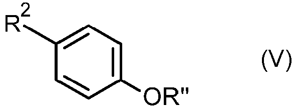
in the presence of a base if R" is hydrogen;
wherein the variables are defined as follows:
R² is F or Cl;
R" is hydrogen, N(C₁-C₈-alkyl)₄ cation or an alkali metal cation.

## Patentansprüche

1. Ein Verfahren zur Herstellung der Grignard-Verbindung der Formel (I) 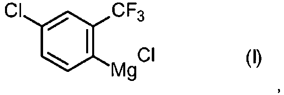 bestehend aus den folgenden Schritten:
(i) reaktion einer Verbindung der Formel (A) 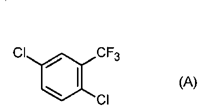 mit Magnesium in Anwesenheit von LiCI und einem Lösungsmittel.

2. Der Prozess von Claim 1, bei dem das Mg aktiviert wird.

3. Der Prozess von Anspruch 2, bei dem der Magnesiumaktivator 2-Brompropan ist.

4. Der Prozess eines der Ansprüche 1 bis 3, bei dem das Lösungsmittel aus aliphatischen, alicyclischen oder aromatischen Kohlenwasserstoffen, Ethern und Polyethern von Ethylenoxid und/oder ProPylenoxid.

5. Das Verfahren bei jedem der Ansprüche 1 bis 4, bei dem das Lösungsmittel aus Hexan, Heptan, Cyclohexan, Tetrahydrofuran (THF), 2-Methyltetrahydrofuran, 1,4-Dioxan, Diethylether, Methyl-tert-Butylether (MTBE), Toluol, m-Xylen, O-Xylen, p-Xylol oder einer beliebigen Mischung daraus ausgewählt wird.

6. Der Prozess eines der Ansprüche 1 bis 5, bei dem das Lösungsmittel THF ist.

7. Ein Verfahren zur Herstellung von Verbindungen der Formel (II) 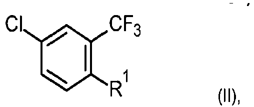 woin:
R1 wird ausgewählt aus - CR^{a}R^{b}OH, -C(O) R^{c}, -B(OH)2, B(OC₁-C₄-Alkyl)₂
R^{a} und R^{b} werden unabhängig voneinander aus Hor C₁-C₄-Alkyl oder Phenyl ausgewählt, wobei
Phenyl kann unersetzt sein oder 1, 2 oder 3 Substituenten tragen, die aus Halogen oder C₁-C₆-Alkyl ausgewählt werden; oder
R^{a} und R^{b} bilden zusammen mit dem C-Atom, an das sie befestigt sind, einen C₃-C₆-cy-Kloakylring;
Re wird aus H, OH oder C₁-C₄-Alkyl oder Phenyl ausgewählt, wobei Phenyl unersetzen oder tragen 1, 2 oder 3 Substituenten, die aus Halogen oder C₁-C₆-Alkyl ausgewählt werden; bestehend aus folgenden Schritten:
(i) Vorbereitung der Verbindung der Formel (I) gemäß dem Prozess
Wie in Anspruch 1 beschrieben, 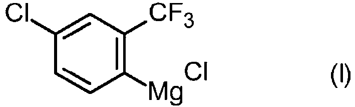
(ii) wobei die Verbindung der Formel (I) mit einem Elektrophilen reagiert, wobei der Elektrophile aus ausgewählt wird
(E1) RaC(O)Rb,
(E2) WC(O)X,
(E3) B(OC1-C4-Alkylh,
(E4) CO2, 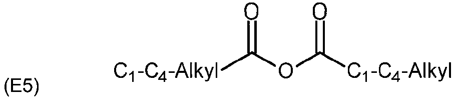
oder
(E6) RcCN
wobei
R^{a} und R^{b} werden unabhängig voneinander aus Hor C₁-C₄-Alkyl oder Phenyl ausgewählt, wobei Phenyl unsubstituiert sein kann oder 1, 2 oder 3 Substituenten aus Halogen oder C₁-C₆-Alkyl tragen kann; oder
R^{a} und R^{b} bilden zusammen mit dem C-Atom, an das sie befestigt sind, einen C₃-C₆-cy -Kloakylring;
R^{c} wird aus H, C₁-C₄-Alkyl oder Phenyl ausgewählt, wobei Phenyl unsubstituiert sein kann oder 1, 2 oder 3 Substituenten aus Halogen oder C₁-C₆-Alkyl tragen kann;
X wird aus Halogen oder -N(C₁-C₄-alkyl)₂, -N(C₁-C₄-alkyl)-O-( C₁-C₄-alkyl) ausgewählt, N-Morfolin.

8. Der Prozess des Anspruchs 7, bei dem der Elektrophile aus ausgewählt wird
(E2-1) RcC(O)X,
wobei X ein Halogen darstellt,
(E2-2) R^{c}C(O)N(C₁-C₄-alkyl)-O-(C₁-C₄-alkyl), 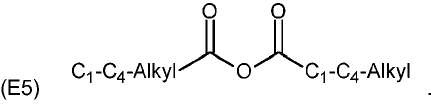
oder
(E6) R^{cCN}

9. Das Verfahren des Anspruchs 8, bei dem das Elektrophil aus Acetylchlorid (AcCl) oder Essigsäureanhydrid (Ac2O) ausgewählt wird.

10. Der Prozess eines der Ansprüche 7 bis 9, bei dem die Reaktion in Anwesenheit eines Cu-Katalysators durchgeführt wird.

11. Ein Verfahren zur Herstellung einer Formelverbindung (III) 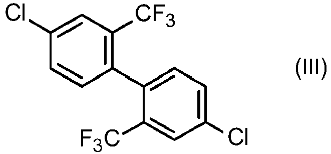 bestehend aus den folgenden Schritten:
(i) Vorbereitung der Verbindung der Formel (I) gemäß dem in Anspruch 1 beschriebenen Verfahren, 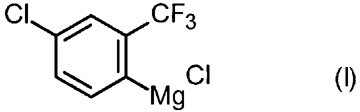
(iii) wobei die Verbindung der Formel (I) dimerisiert wird.

12. Der Prozess des Anspruchs 11, bei dem die Dimerisierung in Anwesenheit eines Katalysator.

13. Das Verfahren von Anspruch 11 oder 12, bei dem die Reaktion in Anwesenheit eines terminalen Oxidationsmittels aus Dioxygen, einem Diaziridinon oder 1,2-Dihaloethan ausgewählt wird.

14. Ein Verfahren zur Herstellung einer Verbindung der Formel (IV) 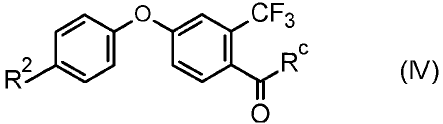 wobei
R^{c} ist C₁-C₄-Alkyl;
R² ist für Cl;
bestehend aus den folgenden Schritten:
(i) Vorbereitung der Verbindung der Formel (I) gemäß dem in Anspruch 1 beschriebenen Verfahren, 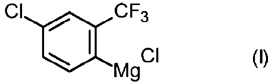
(ii) Herstellung der Verbindung der Formel (II-A) gemäß dem im Anspruch beschriebenen Verfahren 8, 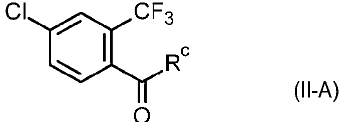 wherein R^{c} is C₁-C₄-alkylj
(iv) reagierende Verbindung (II-A) mit einer Phenolableitung der Formel (V) 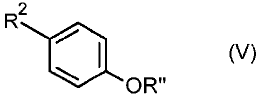
in Anwesenheit einer Basis, falls R"
Wasserstoff ist; wobei die Variablen wie
folgt definiert sind:
R² ist für Cl;
R" ist Wasserstoff, N(C₁-C₈-alkyl)₄-Kation oder ein Alkalimetall-Kation.

## Revendications

1. Un procédé de préparation du composé de Grignard de la formule (I) 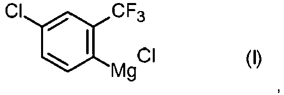 comprenant les étapes suivantes :
(i) réaction d'un composé de formule (A) 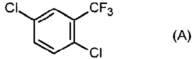 avec du Mg en présence de LiCl et d'un solvant.

2. Le processus de la revendication 1, dans lequel le Mg est activé.

3. Le processus de la revendication 2, où l'activateur de magnésium est le 2-bromopropane.

4. Le procédé de l'une des revendications 1 à 3, dans lequel le solvant est sélectionné parmi des hydrocarbures aliphatiques, alicycliques ou aromatiques, éthers et polyethers d'oxyde d'éthylène et/ou pro-oxyde de pylène.

5. Le procédé de l'une des revendications 1 à 4, dans lequel le solvant est sélectionné parmi l'hexane, l'heptane, le cyclohexane, le tétrahydrofurane (THF), le 2-méthyl-tétrahydrofurane, le 1,4-dioxane, l'éther diéthylique, le méthyl-tert-butyléther (MTBE), le toluène, le m-xylène, l'o-xylène, le p-xylène ou tout autre mélange de ceux-ci.

6. Le procédé de l'une des revendications 1 à 5, dans lequel le solvant est THF.

7. Un procédé de préparation des composés de formule (II) 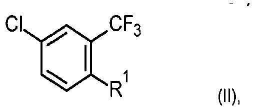 dans lequel:
R1 est sélectionné parmi -CR^{a}R^{b}OH, -C(O)R^{c}, -B(OH)2, B(OC₁-C₄-Alkyl)₂
R^{a} et R^{b} sont sélectionnés indépendamment de Hor C₁-C₄-alkyl ou phényle, où le phényle peut être non substitué ou porter 1, 2 ou 3 substituants sélectionnés parmi des halogènes ou du C₁-C₆-alkyle ; ou
R^{a} et R^{b}, ainsi que l'atome C auquel ils sont attachés, forment un anneau C₃-C₆-cy-cloalkyl ;
R^{c} est sélectionné à partir de H, OH ou C₁-C₄-alkyle ou phényl, où le phényl peut être non-substitué ou portent 1, 2 ou 3 substituts sélectionnés à partir d'halogènes ou
de C₁-C₆-alkyle ; comprenant les étapes suivantes :
(i) préparation du composé de formule (I) selon le procédé Comme décrit dans la revendication 1, 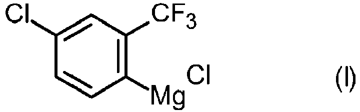
(ii) réagir le composé de formule (I) avec un électrophile, où l'électro-phile est sélectionné parmi
(E1) RaC(O)Rb,
(E2) WC(O)X,
(E3) B(OC1-C4-Alkylh,
(E4) CO2, 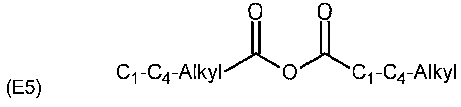
ou
(E6) R^{c}CN
où
R^{a} et R^{b} sont sélectionnés indépendamment de Hor C₁-C₄-alkyl, ou phényl, où le phényle peut être non substitué ou porter 1, 2 ou 3 substituants sélectionnés à partir d'halogènes ou de C₁-C₆-alkyle ; ou
R^{a} et R^{b}, ainsi que l'atome C auquel ils sont attachés, forment un anneau C₃-C₆-cy-cloalkyl ;
R^{c} est sélectionné parmi H, C₁-C₄-alkyle ou phényle, où le phényle peut être non sous-tué ou porter 1, 2 ou 3 substituants sélectionnés à partir d'halogènes ou de C₁-C₆-alkyle ;
X est sélectionné à partir d'halogènes ou -N(C₁-C₄-alkyl)₂, -N(C₁-C₄-alkyl)-O-( C₁-C₄-alkyle), N-morpholine.

8. Le processus de la revendication 7, dans lequel l'électrophile est sélectionné parmi
(E2-1) RcC(O)X, où X représente un halogène,
(E2-2) R^{c}C(O)N(C₁-C₄-alkyl)-O-(C₁-C₄-alkyl), 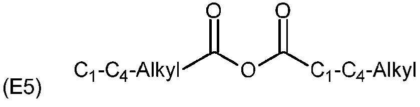
.ou
(E6) R^{cCN}

9. Le procédé de la revendication 8, dans lequel l'électrophile est sélectionné à partir de chlorure d'acétyl (AcCl) ou d'anhydride d'acide acétique (Ac2O).

10. Le processus de l'une des revendications 7 à 9, où la réaction est réalisée en présence d'un catalyseur Cu.

11. Un procédé de préparation d'un composé de formule (III) 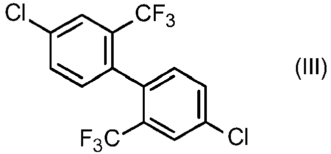 comprenant les étapes suivantes :
(i) la préparation du composé de formule (I) selon le processus
décrit dans l'affirmation 1, 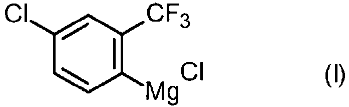
(iii) dimérisant le composé de la formule (I).

12. Le processus de la revendication 11, dans lequel la dimérisation est réalisée en présence d'un catalyseur.

13. Le procédé de la revendication 11 ou 12, dans lequel la réaction est effectuée en présence d'un agent oxydant terminal sélectionné à partir de dioxygène, de diaziridinone ou de 1,2-dihaloéthane.

14. Un procédé de préparation d'un composé de formule (IV) 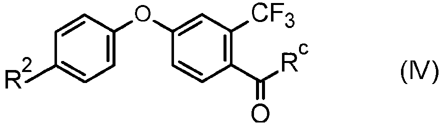 où
R^{c} est C₁-C₄
alkyle ;
R² est Pour CI ;
comprenant les étapes suivantes :
(i) la préparation du composé de formule (I) selon le processus décrit dans l'affirmation 1, 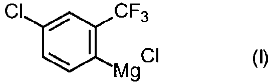
(ii) préparation du composé de formule (II-A) selon le processus décrit dans la revendication 8, 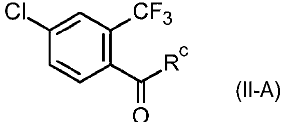 wherein R^{c} is C₁-C₄-alkyl,
(iv) composé réactif (II-A) avec une dérivée phénolienne de formule (V) 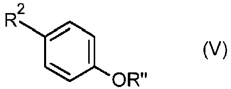
en présence d'une base si R » est de
l'hydrogène ; où les variables sont définies
comme suit :
R² est F our CI ;
R" est un cation hydrogène, N(C₁-C₈-alkyl)₄ ou un cation de métaux alcalins.
